(19) Europäisches Patentamt
European Patent Office
Office européen des brevets

(11) EP 0 523 813 B1

(12) **EUROPEAN PATENT SPECIFICATION**

(45) Date of publication and mention
of the grant of the patent:
**27.11.1996 Bulletin 1996/48**

(51) Int Cl.[6]: **C07K 5/075**, A23L 1/236

(21) Application number: **92202213.2**

(22) Date of filing: **17.07.1992**

(54) **Method for crystallizing alpha-L-aspartyl-L-phenylalanine methyl ester**

Verfahren zur Kristallisation von Alpha-L-Aspartyl-L-Phenylalaninmethylester

Procédé de cristallisation de l'alpha-l-aspartyl-l-phénylalanine ester méthylique

(84) Designated Contracting States:
**AT BE CH DE DK ES FR GB GR IT LI LU MC NL PT SE**

(30) Priority: **19.07.1991 JP 203703/91**

(43) Date of publication of application:
**20.01.1993 Bulletin 1993/03**

(73) Proprietor: **HOLLAND SWEETENER COMPANY V.O.F.**
**NL-6212 XW Maastricht (NL)**

(72) Inventors:
• **Okajima, Kengo**
  **Shinnanyo-shi, Yamaguchi, 746 (JP)**
• **Vermijs, Winfried Johannes Wouterus**
  **6164 BE Geleen (NL)**
• **Murakami, Tsuguo**
  **Shinnanyo-shi, Yamaguchi, 746 (JP)**

• **Egashira, Hidetaka**
  **Shinnanyo-shi, Yamaguchi, 746 (JP)**
• **Takeshima, Yukio**
  **Yamaguchi, 746 (JP)**

(74) Representative:
**den Hartog, Jeroen Hendrikus Joseph et al**
**Octrooibureau DSM**
**Postbus 9**
**6160 MA Geleen (NL)**

(56) References cited:
**EP-A- 0 399 605         EP-A- 0 405 273**

Remarks:
The file contains technical information submitted
after the application was filed and not included in this
specification

## Description

This invention relates to a method for crystallizing alphaLaspartyl-L-phenylalanine methyl ester.

[BACKGROUND OF THE INVENTION]

Alpha-L-aspartyl-L-phenylalanine methyl ester (hereinafter referred to as "APM") is a substance which is expected to find wide application as a low-calorie sweetener because of its sweetness of good quality, which is about 200 times as sweet as sugar.

APM can be synthesized by various methods. Among industrial production processes for producing APM are included the one in which an N-protected aspartic acid is condensed with phenylalanine methyl ester in the presence of an enzyme, followed by the elimination of the protecting group (US-A-4,282,721), and the one in which an N-protected aspartic acid anhydride is condensed with phenylalanine methyl ester in an organic solvent, and the protecting group is eliminated in accordance with a conventional method (US-A-3,786,039).

In any process, the crystallization step is indispensable to produce the final product by isolating APM from the reaction mixture. The crystallization step is ordinarily carried out by (i) precipitating APM crystals through cooling of (a) an APM solution obtained via synthesis and purification steps or (b) an APM solution prepared by redissolving a crude APM product into water, an organic solvent or an aqueous system containing organic solvent, (ii) subjecting the precipitated crystals to solid-liquid separation and dewatering by use, e.g., of a centrifugal separator and then (iii) drying the dewatered crystals to give the final product.

Such a crystallization through cooling is usually carried out by using a crystallizer provided with a stirrer and a cooling/heat-transfer surface or a crystallizer provided with a heat-exchanger of external circulation type. There is also known a method in which crystallization is effected through conductive heat transfer without forced flow so as to improve the crystal properties of APM (EP-B-91. 787).

[PROBLEMS TO BE SOLVED BY THE INVENTION]

However, when APM is crystallized through cooling in a crystallizer which utilizes forced flow of, e.g., ordinary stirring or external circulation, crystals obtained are relatively poor in solid-liquid reparability or dewatering properties. Such crystals readily adhere to the cooling/heat-transfer surface and may generate so-called scale, which must be removed at regular intervals with interruption of crystallizing operation since it deteriorates the efficiency of heat-transfer. Since the crystals are fine and have a high water content, also various problems arise during handling. For example, in the drying step, there results a product with undesirably high impurity because of the amount of mother liquor containing impurities attached to the APM crystals. The dry product also contains many fine particles or may heavily scatter in the form of fine powders.

In order to avoid such problems, the above-mentioned EP-B-91. 787 proposes a crystallizing method in which an aqueous APM solution is cooled through conductive heat transfer to form a pseudo solid phase without forced flow of mechanical stirring or the like, followed by further cooling of the system, where required.

By the above method, there can be obtained APM crystals having improved filtering and dewatering properties. However, the method is poor in efficiency since the cooling effected through conductive heat transfer with no forced flow and continued even after the formation of the pseudo solid phase. Because of this, it is required to use a plurality of small crystallizers, to perform cooling over a long period of time, or to use a special crystallizer as shown in the above-mentioned patent.

[OBJECT OF THE INVENTION]

It is an object of this invention to provide a method for crystallizing APM by cooling which enables to obtain big, strong APM crystals that exhibit good filtering and drying properties and hence are advantageous in production, quality and handling.

[MEANS TAKEN TO SOLVE THE PROBLEM]

In view of the above, the inventors have conducted intensive investigations to improve the properties of APM crystals. As a result, it has been found surprisingly that big, strong APM crystals can be obtained when APM is crystallized by cooling an APM solution through direct contact with ice, and that the crystallization can be completed within a short period of time. This invention has been completed based on the above finding.

Accordingly, this invention provides a method for crystallizing α-L-aspartyl-L-phenylalanine methyl ester, which comprises that a solution of α-L-aspartyl-L-phenylalanine methyl ester is cooled through direct contact with ice to a temperature in the range of 0 to 30°C.

This invention will further be explained hereinbelow in detail.

In the method of this invention, any solution of APM can be used including aqueous solutions of APM, a solution of APM in water miscible organic solvent and a solution of APM in a water-containing and water miscible organic solvent. However, the use of an APM solution of which the solvent substantially consists of water is advantageous with regard to the growth of APM crystals, operability and handling.

In such aqueous solutions, generally the amount of water is at least 85% by weight of the solvent.

There are no particular restrictions on the shape or size of the ice to be used. Use of ice that weighs 100 g or less a piece is advantageous with regard to the dissolution rate of the ice (and the cooling rate by the ice), the cost for producing the ice, handling, etc.

The shape of the ice can be cubic, spherical or any other shape. It is also possible to use a crystallizer having a layer of ice on its bottom and/or walls, which may be prepared by freezing water on the bottom and/or walls of the crystallizer.

In this invention usually ice, which has been prepared from water, is used. It is also possible to use ice prepared from a dilute aqueous APM solution. In the latter case, ice is prepared from an aqueous solution preferably containing APM at a concentration of 2% by weight or less.

The amount of ice to be used is determined by the desired quantity of APM precipitated and/or by the desired temperature of the APM solution. The quantity of APM precipitated and the final temperature resulting from direct cooling with ice can be calculated, taking into consideration the solubility of APM at a particular temperature.

When ice is used for cooling, ice melts into water and lowers the concentration of APM. However, the decrease of the concentration of APM is small since the cooling capacity of ice is large, and hence the temperature of an APM solution can be effectively lowered already by a small quantity of ice. Because of this, the quantity of APM precipitated normally increases with an increase in the quantity of ice used. On the other hand, a higher temperature can be advantageous for the growth of APM crystals. In view of these factors, it can be preferred to cool an APM solution to 0 to 30°C, more preferably to 3 to 20°C.

In combination with the direct cooling with ice, conventional cooling can also be utilized, e.g., indirect cooling using cooling media like cold water or brine.

The direct cooling of APM solution with ice can be effected by adding ice to the solution or vice versa. It is also possible to charge both ice and an APM solution simultaneously into a crystallizer.

The crystallization according to this invention can be conducted either by a continuous method or by a batch method. The latter is advantageous with regard to operation.

The contacting of an APM solution and ice may take place with or without stirring. Stirring, if any, should be effected gently. It is, however, preferred not to stir, if largest crystals are desired.

When an APM solution is contacted with ice, the temperature of the solution decreases, and crystals of APM precipitate. The thus obtained crystals are big and strong. Although the size of the crystals varies depending on conditions, they are usually in the form of rod-like bars or columns having a length of 50 to 1,000 $\mu$m or above and a width of 5 to 50 $\mu$m or above.

The precipitation of APM continues until the concentration of APM decreases almost to its solubility at the temperature. However, the period for this precipitation is short, and precipitation terminates within 10 to 120 minutes even when stirring is not effected. In the case where stirring is not effected, there may be some difference in the temperature between the upper and lower parts of the crystallizer which will have more or less supersaturation. Although such difference in supersaturation does not matter, it is preferred to resolve it by means of stirring at the time when the precipitation of APM has almost completed (which is almost identical with the time when the ice has almost melted). After the direct cooling with ice, the contents of the crystallizer may be cooled further indirectly by cold water, brine, or the like. A temperature in the range of 3 to 10°C is preferred.

In indirect cooling, accordingto the state of the art, the precipitation of APM starts at positions near to the cooling/heat-transfer surface. On the other hand, using direct cooling with ice according to this invention, the precipitation begins at positions a little remote from the ice and spreads to the entire solution. The method of this invention therefore is substantially free from the problem of scaling. This is another characteristic feature of this invention.

There are no particular restrictions on the pH of APM solution to be used in this invention. It can, however, be advantageous for the growth of crystals to use an APM solution of a pH of from 3 to 6, in particular from 4 to 5.

The slurry of APM crystals obtained can be filtered either by a batch method or by a continuous method, and a high dewatering percentage can be attained within a short period of time. For the filtration, there can be used any filtering apparatuses ordinarily employed in the industry, including, e.g., centrifugal separators, filter presses, belt filters and drum filters. If desired, the wet cake obtained by the filtration can be washed with water or with a solution of APM. The washing is easy. The quality of the product can be improved by washing since the mother liquor attached to the cake is further removed by the washing. The wet cake obtained can be dried either as it is or after being granulated. For the drying all kinds of drying equipment can be used, e.g., a flash dryer, a fluidized-bed dryer or a rotary dryer.

It is not possible to clearly explain the reasons why big and strong APM crystals having good filtering properties are obtained when APM is crystallized through direct cooling with ice. However, based on careful observation on the state of crystallization of APM crystals, the inventors presume as follows:

Immediately after the addition of ice to an APM solution (or vise versa), the ice starts to melt and the temperature of the APM solution decreases. Although the temperature of the APM solution is lower around the ice, the precipitation of APM starts in areas a little remote from the ice. APM crystals so precipitated are big and strong and in the form of rod-like bars or columns. When ice melts, water is formed, which more or less decreases the concentration of APM. Thus, places nearer to ice become lower in the concentration of APM.

That is to say, positions nearer to the ice are lower in temperature of the APM solution, as well as in concentration of APM. On the other hand, with higher APM concentration and/or lower temperature, crystals of APM precipitate earlier and grow faster. By the direct cooling with ice, an appropriate relationship between temperature and APM concentration is attained. As a result, an appropriate number of APM nuclei is formed and APM crystals grow at a high growth rate, thus giving big and strong APM crystals.

In experiments performed by the inventors, APM solutions were cooled directly by using dry ice in place of ice. In such case, extremely fine APM crystals were formed, which were very poor in filtering properties. Moveover, the temperature of APM solution dropped rapidly near the dry ice. Because of this, the precipitation of APM started on or near the surface of dry ice and many crystal nuclei were formed at an extremely high rate. This is presumably the reason why fine APM crystals were formed with no substantial growth in diameter of precipitated crystals.

In another experiment made by the inventors, APM solutions were indirectly cooled with ice by using polyethylene bags, each containing a piece of cubic ice. In this case, precipitation of APM crystals started on the surface of the polyethylene bags, and the rate of precipitation was high. In addition, APM crystals precipitated were small. This also seems to be related to the temperature and the APM concentration.

Big and strong APM crystals having good filtering properties can therefore be obtained only by directly cooling an APM solution with ice, which melts into water to decrease more or less the concentration of APM, as is shown by this invention.

It should be noticed that in Example 5 of EP-A-399.605 a hot solution of APM is added to cold water. The properties of the crystals obtained according to that experiment, however, are less favorable than the properties of the crystals obtained according to the present invention.

[ADVANTAGEOUS EFFECTS OF THE INVENTION]

As is described hereinabove, strong APM crystals having a big crystal size, in particular strong APM crystals well grown in diameter, are obtained within a short period of time in accordance with the method of this invention. As a result, many important advantages set forth below are attained.

(1) The precipitation of crystals is completed by a simple operation within a short period of time due to direct cooling. Because of this, productivity is improved, operation is easier, and economic efficiency is high;
(2) The slurry of APM crystals obtained can be filtered and washed easily, and hence a wet cake of APM crystals having a low water content and containing less impurities is obtained within a short period of time;
(3) Since the quantity of water attached to the wet APM cake is small, the cake can be easily dried at a low temperature with less drying energy in a shorter period of time, to give the final product. In addition, a high-quality APM is obtained since it does not deteriorate in quality upon drying. Furthermore, the cake suffers less from the generation of dust or fine powder in the drying step, which is advantageous with regard to operation;
(4) The dried final product suffers less from the generation of dust or fine powders. This is highly advantageous in its handling; and
(5) If desired, the APM crystals obtained in accordance with this invention can be subjected to wet crushing to give fine crystals, which can be used as seed crystals in a conventional APM precipitation process to improve the crystals to be precipitated.

[Examples]

This invention will further be explained by way of examples. It should, however, be noted that this invention is by no means limited to these.

In the following examples, the filtering rate of the crystals was measured in the following manner.

By using a suction filter (leaf tester) fitted with a polypropylene cloth filter having a permeability of air of 5 ml/cm$^2$·sec (12 mm H$_2$O), 500 ml of slurry containing precipitated APM crystals was filtered at -400 mmHg, whereby the slurry was poured carefully onto the filter so that the filtering could be performed continuously with no drying up of slurry on the filter cloth throughout the operation. The filtering rate was 1 0 calculated from the period of time lapsed from the start to the completion of the filtering (the filtering was considered complete when the solvent of the slurry no more remained on the filter cloth) and the quantity of filtrate resulted from the filtering. The specific resistivity ($\alpha$) was calculated from the change in the volume of filtrate with the lapse of time, in accordance with the following formula:

$$\frac{t}{V} = \frac{\alpha \cdot \eta \cdot C}{2 . \Delta P . A^2} V + \frac{Rm . \eta}{\Delta P . A} \qquad (S/m^3)$$

wherein:

t =      filtering time (s)
V =      Volume of filtrate (m$^3$)
$\alpha$ =      specific resistivity of filetered cake (m/kg)
$\eta$ =      Dynamic viscosity of filtrate (Pa.s)
$\Delta P$ =      Differential pressure of filtrate across the filter plus the cake (Pa)
C =      Weight of crystals precipitated per unit volume of filtrate obtained (kg/m$^3$)
Rm =      Resistivity of filter ($l$/m)

A = filtering area (m$^2$)

in which :

P = 53,329 Pa
A = 1/127 m$^2$

## Example 1

Into a glass flask (internal volume: 2.0 $l$), 1 kg of aqueous 3.5 wt% APM solution (60°C) was charged and the pH of the solution was adjusted to 4.5 with aqueous 1N sodium hydroxide. Then, 500 g of ice cubes (3 cm x 3 cm x 2.5 cm), prepared from pure water, was added thereto at once. About 5 seconds after the completion of the addition of the ice cubes, APM crystals started to precipitate in lower areas a little remote from the ice layer. Fifty-five (55) minutes later, a blade-type stirrer was inserted and rotated to stir the slurry formed in the flask when the ice had melted almost completely and the precipitation of APM had almost ceased. The rotation of the stirrer was smooth and no scale was formed. The temperature of the slurry was 16.5°C, and APM crystals contained in the slurry were in the form of rod-like bars or columns having a width of 5 to 35 μm and a length of 100 μ or above. The filtering rate of the slurry was 439 $l$/m$^2$·min.; the specific resistivity (α) was 3.44 x 10$^9$ m/kg, and filtering was quite smooth.

## Example 2

Into a glass flask (internal volume: 2.0 $l$), 400 g of ice cubes, identical with the one used in Example 1, was charged. Then, 1 kg of aqueous 4.0 wt% APM solution (60°C) of a pH of 4.5 was added thereto at once. About 4 seconds after the addition of the aqueous APM solution, APM crystals started to precipitate in lower areas a little remote from the ice layer.

Forty (40) minutes later, a blade-type stirrer was inserted and rotated to stir the slurry formed in the flask when the ice had melted almost completely and the precipitation of APM crystals had almost ceased. The rotation of the stirrer was smooth and no scale was formed. The temperature of the slurry was 19.5°C, and APM crystals contained therein were in the form of rod-like bars or columns having a width of 5 to 30 μm and a length of 100 μm or above. The filtering rate was 427 $l$/m$^2$·min.; the specific resistivity (α) was 4.27 x 10$^9$ m/kg, and filtering was quite smooth.

## Example 3

Into a glass flask (internal volume: 2.5 $l$), fitted with an external cooling jacket, 643 g of crushed ice produced by crushing the same ice cubes as used in Example 1 by using an ice crusher (Rakuwari ASC-2000, manufactured by Tiger Vacuum Bottle Co., Ltd.), was charged. Then, 1,360 g of aqueous 4.0 wt% APM solution (60°C) of a the pH of 4.5 was added thereto at once.

Several seconds after the addition of aqueous APM solution, APM crystals started to precipitate in lower areas a little remote from the ice layer.

Thirty (30) minutes later, a blade-type stirrer was fitted to the flask and rotated when the ice had melted almost completely and the precipitation of APM crystals had almost ceased. The rotation of the stirrer was smooth, and no scale was formed. The temperature of the slurry was 16.5°C, and APM crystals contained in the slurry were in the form of rod-like bars or columns having a width of 3 to 25 μm and a length of 100 μm or above. Thereafter, the flask was cooled by circulating cold water through the outer jacket of the flask, during which the slurry was stirred by rotating the stirrer at 300 r.p.m.

The cooling was terminated at the time when the temperature of the slurry had dropped to 5°C (which was 50 minutes after the start of the cooling), and the slurry was taken out. Although a small quantity of fine crystals was formed during the cooling, almost all the APM crystals remained in the form of rod-like bars or columns with slight additional growth. The filtering rate of the slurry was 359 $l$/m$^2$·min., specific resistivity (α) was 7.42 x 10$^8$ m/kg, and filtering was quite smooth. No scale was formed on the wall of the flask.

## Example 4

In this example, a glass-made cylindrical crystallizer having an inner diameter of 5 cm and a length of 53 cm and fitted with a cooling jacket was used. At first, a refrigerant of -7°C was circulated through the jacket and water, cooled to ca. 2°C, was charged into the crystallizer. Fifty (50) minutes later, water which remained unfrozen was discharged. By this operation, a layer of ice about 5 mm thick was formed on the wall of the crystallizer. The amount of frozen water was 330 g (calculated from the quantity of water charged and the quantity of water discharged). Immediately after the discharge, 666 g of aqueous 3.5 wt% APM solution (60°C) was charged into the crystallizer. Several seconds later, APM crystals started to precipitate in areas a little remote from the layer of ice. Fifteen (15) minutes later, the APM slurry formed therein was discharged by gravity from the bottom of the crystallizer when the ice had melted almost completely and the precipitation of APM had almost ceased. The discharge of the slurry was smooth, and no scale was generated.

The temperature of the slurry was 15°C, and APM crystals contained in the slurry were in the form of rod-like bars or columns having a width of 5 to 20 μm and a length of 100 μm or above. The filtering rate of the slurry was 419 $l$/m$^2$·min.; specific resistivity (α) was 3.70 x 10$9$ m/kg, and filtering was quite smooth.

## Example 5

Into a glass flask (internal volume: 2.0 *l*), 474 g of ice cubes (3 cm x 3 cm x 2.5 cm), prepared from an aqueous 0.5 wt% APM solution, was charged. Then, 1 kg of aqueous 3.5 wt% APM solution (60°C) of a pH of 4.5 was added thereto at once. In 1 to 2 seconds after the addition of the aqueous APM solution, APM crystals started to precipitate.

Eighty (80) minutes later, a blade-type stirrer was fitted and rotated to stir the slurry formed in the flask when the ice had melted almost completely and the precipitation of APM crystals had almost ceased. The temperature of the slurry was 13.5°C, and APM crystals contained in the slurry were in the form of rod-like bars or columns having a width of 2 to 20 μm and a length of 100 μm or above. The filtering rate was 369 $l/m^2$·min.; the specific resistivity ($\alpha$) was 4.94 x $10^9$ m/kg, and filtering was quite smooth.

The APM crystals were a little smaller than those obtained in Example 2.

## Comparative Example 1

Into a glass flask (inter volume; 2.5 *l*) fitted with a stirrer and an outer cooling jacket 2 *l* of aqueous 3.5 wt% APM solution (60°C) of a pH of 4.5 was charged, and the solution was cooled at a rate of 15°C/hour to 10°C, during which the stirrer was rotated at 300 r.p.m. The thus obtained APM crystals had width of 10 μm or less and a length of 30 to 100 μm. The filtering rate of the slurry was 88 $l/m^2$·min.; the specific resistivity ($\alpha$) was 3.24 x $10^{10}$ m/kg, and filtering of the slurry was difficult.

## Comparative Example 2

The procedure of Example 2 was repeated, except that 360 g of dry ice was used instead of ice cubes in a 3 *l* flask. Immediately after the addition of aqueous APM solution (60°C, pH=4.5, 3.5wt%), APM crystals precipitated on the surface of the dry ice, and the resultant slurry turned into a boiling state with sublimation of carbon dioxide gas. Once the surface of the dry ice had been covered with precipitated APM crystals, the cooling of the slurry proceeded only slowly. When the contents of the flask were observed 1.5 hours after the addition of APM solution, a small quantity of dry ice still remained. APM crystals contained in the slurry were in the form of fine crystals having a width of 5 μm or less and a length of 10 to 100 μm.

The slurry was then gradually stirred with the stirrer to allow the remaining dry ice to sublimate. The temperature of the slurry was 20.5°C. The filtering rate was 75 $l/m^2$·min.; the specific resistivity ($\alpha$) was 5.08 x $10^{10}$ m/kg, and filtering was difficult.

## Claims

1. A method for crystallizing alpha-L-aspartyl-Lphenylalanine methyl ester from its solution by cooling characterized in that a solution of alpha-L-aspartyl-Lphenylalanine methyl ester is cooled through direct contact with ice to a temperature in the range of 0 to 30°C.

2. A method according to claim 1, wherein in said solution the amount of water is at least 85% by weight.

3. A method according to claim 1 or claim 2 wherein the ice has been prepared from an aqueous solution containing alpha-Laspartyl-L-phenylalanine methyl ester at a concentration of 2% by weight or less.

4. A method according to any of claims 1 to 3, wherein cooling with ice takes place to a temperature in the range of 3 to 20°C.

5. A method according to any of claims 1 to 4, wherein the pH of the solution containing alpha-L-aspartyl-L-phenylalanine methyl ester, is in the range of 3 to 6, especially in the range of 4 to 5.

6. A method according to any of claims 1 to 5, wherein cooling with ice takes place without stirring.

7. A method according to claim 6, wherein the stirring is started when the ice has almost melted.

8. A method according to any of claims 1 to 7, wherein further cooling takes place by indirect cooling.

## Patentansprüche

1. Verfahren zur Kristallisation von α-L-Aspartyl-L-phenylalaninmethylester aus seiner Lösung durch Abkühlen, dadurch gekennzeichnet, daß eine Lösung von α-L-Aspartyl-L-phenylalaninmethylester durch direkten Kontakt mit Eis auf eine Temperatur im Bereich von 0 bis 30°C abgekühlt wird.

2. Verfahren nach Anspruch 1, bei welchem die Wassermenge in der Lösung zumindest 85 Masse-% beträgt.

3. Verfahren nach Anspruch 1 oder 2, bei welchem das Eis aus einer wässerigen Lösung hergestellt wurde, die α-L-Aspartyl-L-phenylalaninmethylester in einer Konzentration von 2 Masse-% oder weniger enthält.

4. Verfahren nach einem der Ansprüche 1 bis 3, bei welchem das Abkühlen mit Eis auf eine Temperatur im Bereich von 3 bis 20°C stattfindet.

**5.** Verfahren nach einem der Ansprüche 1 bis 4, bei welchem der pH der α-L-Aspartyl-L-phenylalaninmethylester enthaltenden Lösung im Bereich von 3 bis 6, insbesondere im Bereich von 4 bis 5, liegt.

**6.** Verfahren nach einem der Ansprüche 1 bis 5, bei welchem das Abkühlen mit Eis ohne Rühren stattfindet.

**7.** Verfahren nach Anspruch 6, bei welchem das Rühren begonnen wird, wenn das Eis nahezu geschmolzen ist.

**8.** Verfahren nach einem der Ansprüche 1 bis 7, bei welchem ein weiteres Abkühlen durch indirekte Kühlung stattfindet.


**Revendications**

**1.** Procédé de cristallisation de l'ester méthylique de l'alpha-L-aspartyl-L-phénylalanine à partir de sa solution par refroidissement, caractérisé en ce qu'on refroidit une solution de l'ester méthylique de l'alpha-L-aspartyl-L-phénylalanine par contact direct avec de la glace à une température comprise dans l'intervalle de 0 à 30°C.

**2.** Procédé selon la revendication 1, dans lequel dans ladite solution, la quantité d'eau est d'au moins 85 % en poids.

**3.** Procédé selon la revendication 1 ou 2, dans lequel on a préparé la glace à partir d'une solution aqueuse contenant l'ester méthylique de l'alpha-L-aspartyl-L-phénylalanine à une concentration de 2 % en poids ou moins.

**4.** Procédé selon l'une quelconque des revendications 1 à 3, dans lequel le refroidissement par la glace se déroule à une température comprise dans l'intervalle de 3 à 20°C.

**5.** Procédé selon l'une quelconque des revendications 1 à 4, dans lequel le pH de la solution contenant l'ester méthylique de l'alpha-L-aspartyl-L-phénylalanine est compris dans l'intervalle de 3 à 6, en particulier dans l'intervalle de 4 à 5.

**6.** Procédé selon l'une quelconque des revendications 1 à 5, dans lequel le refroidissement par la glace se produit sans agitation.

**7.** Procédé selon la revendication 6, dans lequel l'agitation démarre lorsque la glace a presque fondu.

**8.** Procédé selon l'une quelconque des revendications 1 à 7, dans lequel un refroidissement supplémentaire se produit par refroidissement indirect.